# EUROPEAN PATENT SPECIFICATION

(11) **EP 1 827 412 B1**
(45) Date of publication and mention of the grant of the patent: **20.11.2013**
(21) Application number: 05814069.0
(22) Date of filing: 09.12.2005
(51) Int. Cl.: A61K 31/19, A61P 25/00

(54) **COMPOSITIONS FOR USE IN SURGERY**
ZUSAMMENSETZUNGEN ZUR VERWENDUNG IN DER CHIRURGIE
COMPOSITIONS DESTINEES A UNE UTILISATION CHIRURGICALE

(30) Priority: 10.12.2004 GB 0427145
(43) Date of publication of application: 05.09.2007
(73) Proprietor: BTG International Limited, London EC4M 7RD (GB)
(72) Inventor: HEAL, David John, Pennyfoot Street, Nottingham NG1 1GF (GB); MARTIN, Keith Frank, Ketocytonyx Inc., London EC4M 7SB (GB)
(74) Representative: Dolan, Anthony Patrick
(86) International application number: PCT/GB2005/004723
(87) International publication number: WO 2006/061624

(56) References cited:
- WO-A-01/19361
- WO-A-2005/107875
- US-A- 5 654 266
- US-A1- 2002 013 339
- SMITH S L ET AL: "KTX 0101: A potential metabolic approach to cytoprotection in major surgery and neurological disorders" CNS DRUG REVIEWS 2005 UNITED STATES, vol. 11, no. 2, 2005, pages 113-140, XP009061052 ISSN: 1080-563X
- MANKES R F ET AL: "Birthweight depression in male rats contiguous to male siblings in utero exposed to high doses of 1,3-butanediol during organogenesis" JOURNAL OF THE AMERICAN COLLEGE OF TOXICOLOGY 1986 UNITED STATES, vol. 5, no. 4, 1986, pages 189-196, XP009061055

## Description

The present invention relates to compounds and compositions that have the effect of modulating mammalian central nervous system activity such as to have beneficial effect in surgical procedures where it is desirable to stabilise the patient.

It is well known that surgical procedures are stressful to patients of all age groups, but especially the young and the elderly (eg see Burkhardt et al, 1997; Ornaque et al, 2000; Pace et al, 2004) and premedication prior to surgery is generally employed to reduce the incidence of anxiety, muscle tenseness and insomnia in such patients (eg see Tolksdorf et al, 1987; Drautz et al, 1991; Ornaque et al, 2000; Frank et al, 2002). Drugs employed as premedicating agents include the benzodiazepines (for their anxiolytic, sedative amnesic and muscle relaxant properties), α₂-adrenoceptor agonists (for their sedative, analgesic, anti-emetic and anaesthetic-sparing effects) and opioid/major tranquiliser combinations therapy (also for their sedative, analgesic, anti-emetic and anaesthetic-sparing effects).

In addition, these agents have therapeutic value in the post-operative situation where they are used predominantly to induce sedation (often in critical care situations) and to reduce post-surgical complications and pain (eg see Martin et al, 2003; Moore et al, 1983; Galasko et al; 1985; Reithmuller-Winzen, 1987; Kulka et al 1996; Oliver et al, 1999; Frank et al, 1999, 2002; Kuchta and Golembiewski et al, 2004). However, polypharmacy can lead to unfavourable drug interactions in these patients, particularly in the geriatric population that can result in serious complications and even death. Thus, there is an opportunity in all types of surgery, ie procedures performed under general or local anaesthesia, for the use of a peri-surgical intervention that will provide "stabilisation" for the patient, ie it will produce sedation, anxiolysis, anaesthetic-sparing and/or analgesia, without the potential liability of drug-drug interactions. In this invention, we have demonstrated that ketogenesis unexpectedly provides such a therapeutic intervention.

It is known that both acute and chronic neurodegenerative states in mammals, eg. man, can be treated by inducing ketosis. Such ketosis can be provided by restriction of diet, eg by starvation or exclusion of carbohydrate, or by administration of ketogenic materials, such as triglycerides, free fatty acids, alcohols (eg butan-1, 3-diol), acetoacetate and (R)-3-hydroxybutyrate and their conjugates with each other and further moieties, eg. esters and polymers of these. Ketogenic materials thus produce a physiologically acceptable ketosis when administered to a patient.

Further therapeutic indications for the application of ketosis include epilepsy, diabetes, dystrophies and mitochondrial disorders. In the case of epilepsy ketogenic diet has been applied in treatment of intractable seizures with some success for many years, although the mechanism by which the seizure suppression is achieved remains uncertain.

Copending patent application WO 2005/107875 by KetoCytonyx describes how ketogenic materials may be used to provide treatment for depression, impaired cognitive function, pain, apoptotic conditions, attention deficit disorder, (ADHD) and related CNS disorder symptoms of one or more of impaired learning, impaired problem solving and impaired planning, impulsiveness and aggression

The present inventors have been studying the mode of action of ketogenic materials in CNS injury and particularly have studied whole mammalian brain electrical activity with a view to understanding more completely its overall effect on functioning brain.

Surprisingly, they have now found that completely unanticipated changes in brain electrical activity are induced by ketosis in man such that it is evident that ketosis has a beneficial effect in all types of surgery where it is necessary or desirable to stabilise the patient to allow the other aspects of the procedure to proceed safely. In the context of the present invention, 'stabilise' particularly provides sedation and/or anaesthetic sparing preferably with anxiolysis and/or analgesia.

Analysis of brain field potentials ("Tele-Stereo-EEG") has been proven to be a very sensitive tool for the characterization of drug effects on the central nervous system (Dimpfel et al., 1986). After administration of a centrally active drug, quantitative changes in the brain field potentials can be considered as a characteristic fingerprint of that particular drug. "Fingerprints" of more than 100 compounds have been obtained including 8 established drug categories, e.g. analgesics, antidepressants, neuroleptics, stimulants, tranquilizers, sedatives and narcotics (particularly general anaesthetics). Different dosages of the same drug cause quantitative changes in electrical power.

This methodology can therefore also demonstrate possible dose response relationships. Direct comparison with specific reference drugs, or by discriminant any possible similarities with established drugs. In general, "fingerprints" show prominent differences for drugs prescribed for different indications and are similar for drugs with similar indication (Dimpfel 2003). Furthermore, the pattern of EEG changes in the rat is a useful tool in predicting possible changes in the EEG power spectrum in humans.

Applying this technique to ketosis, particularly that induced by direct administration of (R)-3-hydroxybutyrate sodium salt, the present inventors have been able to show clear changes in the EEG power spectra in human subjects that are consistent with beneficial clinical effects, ie the provision of sedation, anxiolysis and/or analgesia. These are whole brain effects consistent with beneficial effects suited to application to patients undergoing all types of surgery, particularly major surgery.

The present inventors have now studied the effect of ketosis, in the exemplified case induced by administration of sodium salt of (R)-3-hydroxybutyrate (herein referred to as KTX 0101) as a single intravenous infusion of increasing dose and duration to 3 groups (Parts A, B and C) with 3 cohorts of 3 subjects each in Part A, and 1 cohort in each of Parts B (n=3) and C (n=8). Parts A and B were a partial crossover design and Part C was a crossover design. For details see protocol.

A 17-electrode EEG was recorded pre-dose and at 6, 12 and 24 h during the infusion and 1 and 24 hours following the end of drug administration. Recordings were performed under two physiological conditions, namely with 5 minutes eyes open and eyes closed, respectively. KTX 0101 was administered intravenously at a dose of 300 mg/kg given over 24 hours to 1 cohort (n=8) in a double blind placebo controlled crossover design (Part C of the study).

Analysis of the recorded data revealed that consistent deviations from predrug values were found during and after the infusion which could be attributed to drug application. Whereas delta and theta power (averaged over 15 electrode positions because data collection from 2 electrodes, ie Fz and Pz, was compromised) decreased under placebo conditions (environmental stress due to infusion of an unknown drug) this effect was not observed under active drug conditions. On the contrary increases of electrical power were observed, especially with respect to theta, alpha and beta power. Using a non-parametric statistical test for comparing time dependent changes between placebo and active drug some differences were observed at the recording time of 6, 12 and 24 hours after the beginning of the KTX0101 infusion with respect to theta, alpha1,2 and beta1,2 frequencies. Increased power changes in the delta, theta, alpha1,2 and beta1 frequencies, some highly statistically significant, were also observed 1h and 24h after termination of the KTX 0101 infusion.

Since the electrical power within the theta and beta frequencies increases during the cooling of patients (see Kochs, 1995), these changes may be interpreted as indicative of a cytoprotective action of KTX 0101 in these subjects as hypothermia has powerful cytoprotective actions (Wagner and Zuccarello, 2005; Lasater, 2005; Citerio et al, 2004). Power increases in the beta range of varying degrees have been observed in rats after administration of sedative analgesics, including phenobarbital (barbiturate sedative, analgesic, anxiolytic, muscle-relaxant), diazepam (benzodiazepine sedative, anxiolytic, amnesic, muscle-relaxant), buprenorphine and morphine (opiate, narcotic analgesics) and flupertine (non-opiate analgesic) (see Dimpfel et al, 1986). These drug classes are all used as pre-medications in surgery and as agents to manage post-operative pain as well as other complications (see Tolksdorf et al, 1987; Drautz et al, 1991; Burkardt et al, 1997; Frank et al, 1999, 2002; Ornaque et al 2000). Combined increases in theta, alpha1,2 and beta1,2 power have also been reported to occur in rats after administration of noradrenergic α₂-adrenoceptor agonists, eg metedomidine, guanfacine, clonidine, maxonidine and (-)lofexidine, (see Dimpfel and Schober, 2001). This class of drug has long been employed in the pre-surgical setting for its sedative, analgesic, anti-emetic and anaesthetic-sparing effects and post-surgically to prolong anaesthesia-induced analgesia and to reduce post-operative shivering (see Kulka et al, 1996; Oliver et al, 1999; El-Kerdawy et al 2000; Frank et al, 2002; Akbas et al, 2005). Lastly, combined increases in alpha1,2 and beta1,2 power have been reported to occur in rats after administration of general anaesthetics, eg halothane, desflurane, enflurane and isoflurothane (halogenated gaseous anaesthetics) and propofol (steroidal injectable anaesthetic), (see Dimpfel, 2003). Together, these changes in the EEG power spectra evoked by infusion of KTX 0101, which are present not only during the infusion period, but also for many hours after, indicate that KTX 0101 has the unexpected ability to provide "stabilisation" to patients in the peri-surgical setting by virtue of its sedative, anxiolytic, aneasthetic-sparing and/or analgesic actions. KTX 0101 is not a pharmacological intervention because it produces its beneficial effects by providing a key substrate of physiological, mitochondrial oxidative phosphorylation, and therefore, it will not give rise to serious side-effects or adverse events that arise from drug-drug interactions that can arise with conventional agents, eg barbiturates, benzodiazepines, opiates or α₂-adrenoceptor agonists (see Kuchta and Goleimbiewski, 2004).

It is herein described a method of treating a subject in need of medication as an adjunct to elective surgery, comprising administration of a ketogenic material sufficient to produce a physiologically acceptable ketosis in the patient. This takes the application of ketosis into the field of elective surgery in absence of pre-existing trauma eg. of head or trunk and in addition into general surgery (both urgent and non-urgent). Further it is envisaged that this surgery surprisingly might include removal of tumours, removal of redundant organs such as lymph nodes and appendix, open heart surgery, cosmetic surgery, joint and bone surgery and organ transplantation etc.

Preferably the ketosis is such that ketone bodies in the patients blood as sufficient to elevate electrical power of one or more of the theta, aphal and beta1 frequencies in the patients EEG as compared to control levels.

The ketosis produced is preferably a state in which levels of one or both of acetoacetate and (R)-3-hydroxybutyrate concentrations in the blood of the subject are raised. Preferably the total concentration of these 'ketone bodies' in the blood is elevated above the normal fed levels to between 0.1 and 30mM, more preferably to between 0.3 and 15mM, still more preferably to between 0.5 and 10mM and most preferably to between 3 and 8mM. For the purpose of maximising levels of such compounds in the CNS it is desirable to saturate the transporter through which (R)-3-hydroxybutyrate crosses the blood brain barrier: this occurring at between 3 and 5mM.

The ketogenic material according to the invention is , (R)-3-hydroxybutyrate, its salts, oligomers of (R)-3-hydroxybutyrate and esters of (R)-3-hydroxybutyrate with glycerol or (R)-1, 3-butandiol.

Ketogenic materials are known from the following references as set out in Table 1 below. Doses and formats are as described in the documents identified in the table. Typically the amount of ketogenic material required can be determined by measuring blood levels directly using a meter such as the Medisense Precision Extra (MedisenseInc, 4A Crosby Drive Bedford, MA 01730); BioScanner 2000 (formerly called the MTM BioScanner 1000) from Polymer Technology Systems Inc. Indianapolis, Indiana. In this manner the amount of ketosis derived from a set dose may be ascertained, and that dose iterated to suit the individual.

Typical dose ranges for example might be in the range 5 to 5000mg/kg body weight, particularly for an (R)-3-hydroxybuytrate containing material such as oligomeric (R)-3-hydroxybuytrate or its esters with, eg, glycerol or (R)-butan-1,3-diol, more preferably 30 to 2000mg/kg body weight, most preferably 50 to 1000mg/kg body weight per day. Regular blood levels are more readily attained by dosing using a parenteral line through a catheter and drip feed or by a single bolus injection through a saline line.

For parenteral injection a solution containing 1 to 5000mg/kg per day is supplied, typically being the ketogenic material, eg. (R)-3-hydroxybutyrate in aqueous solution, such as in water or in saline. Such solution for bolus injection may be from 5 to 500mM, preferably 28 to 300mM, concentration for injection or higher concentration as a cincentrate for dilution in a drip. Where the ketogenic material is not water soluble it may be administered as an injectable emulsion such as will be known to those skilled in the art.

The use of the claimed ketogenic material is for the manufacture of a medicament for administration in surgery, whether as premedication or during the course of the surgery. Such surgery is advantageously that which is either elective or general surgery (both urgent and non-urgent), as opposed to that required for a pre-existing trauma as is already taught is treatable in the prior art.

The pharmaceutical composition according to the claims is for use in surgery, the surgery particularly being that which is either elective or general surgery (both urgent and non-urgent), rather than that associated with head or trunk trauma. Surgery to remove a tumour, section of gut or tissue is thus contemplated.

**TABLE 1**

| Material | Type | Reference |
|---|---|---|
| Sodium (R)-3-hydroxy-butyrate | Salt | US 4579955 |
| | | US 4771074 |
| (R)-1,3-butandiol | Metabolic precursor | Gueldry al (1994) Metabolic Brain Discase Vol 9 No2 |
| Acetoacetylbutandiol | Metabolic precursor | US 4997976 |
| | | US 5126373 |
| Dimer and trimer BHB | Metabolic precursor | JP 5009185 |
| | | JP 2885261 |
| Acetoacetyltri-3HB | Metabolic precursor | US 6207856 |
| Mid chain triglyceride | Metabolic precursor | WO 01/82928 |
| Triolide | Metabolic precursor | WO00/15216 |
| | | WO00/04895 |
| BHB-triglyceride | Metabolic precursor | US 5420335 |
| | | US 6306828 |
| BHB multimers | Metabolic precursor | WO00/04895 |

| | | |
|---|---|---|
| BHB indicates β-hydroxybutyrate | | |

The present invention will now be described by way of the following Examples and Figures.

### FIGURES

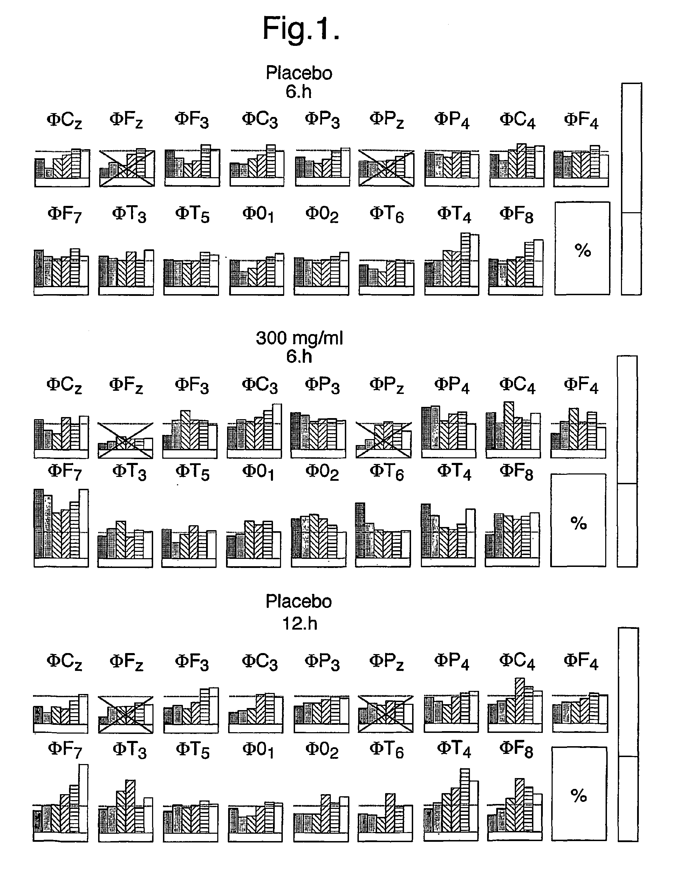

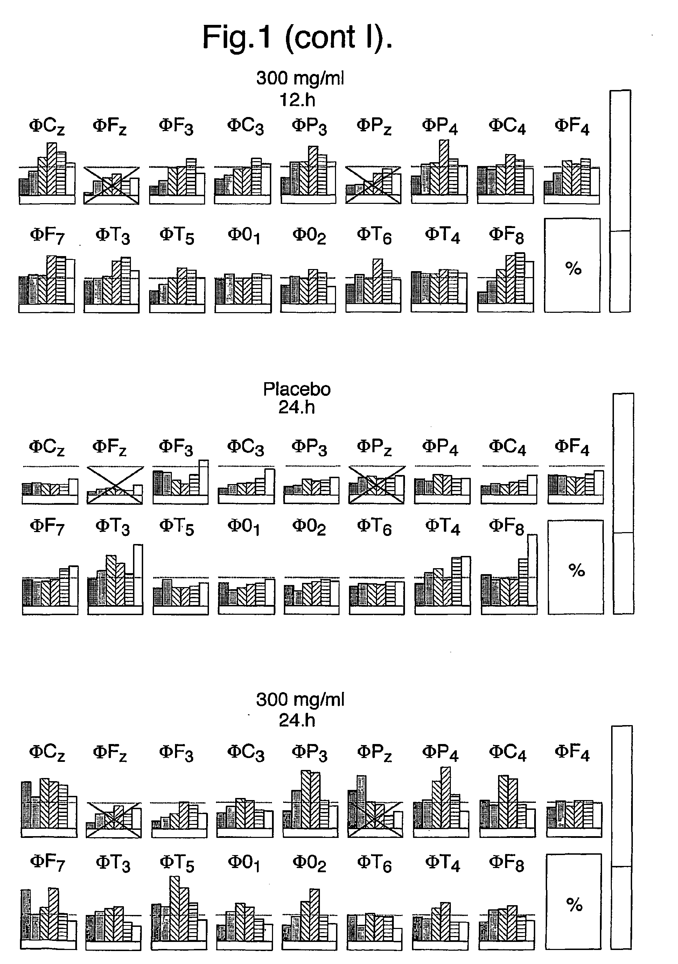

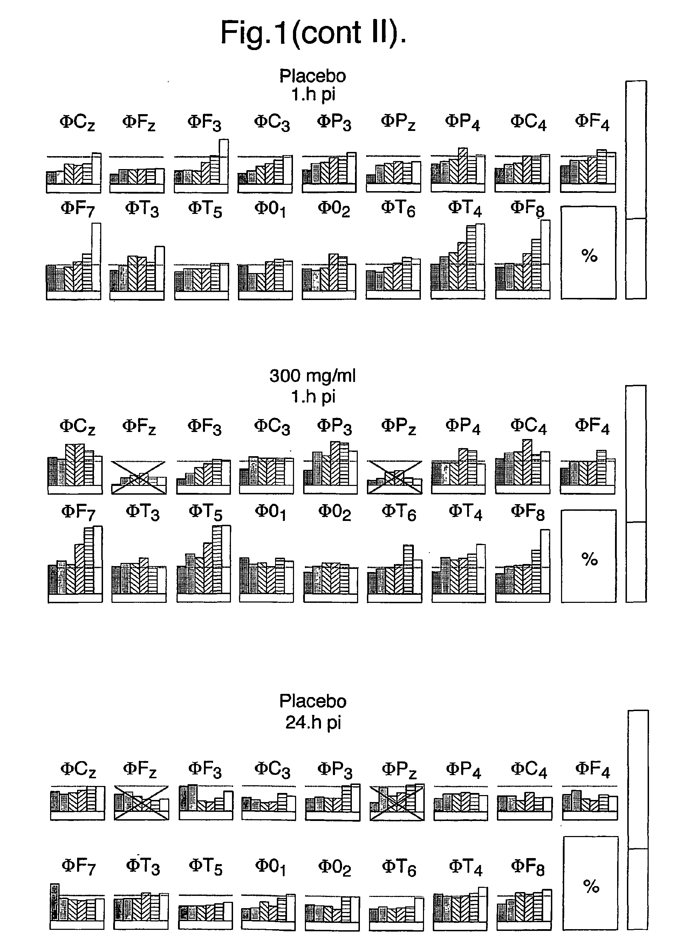

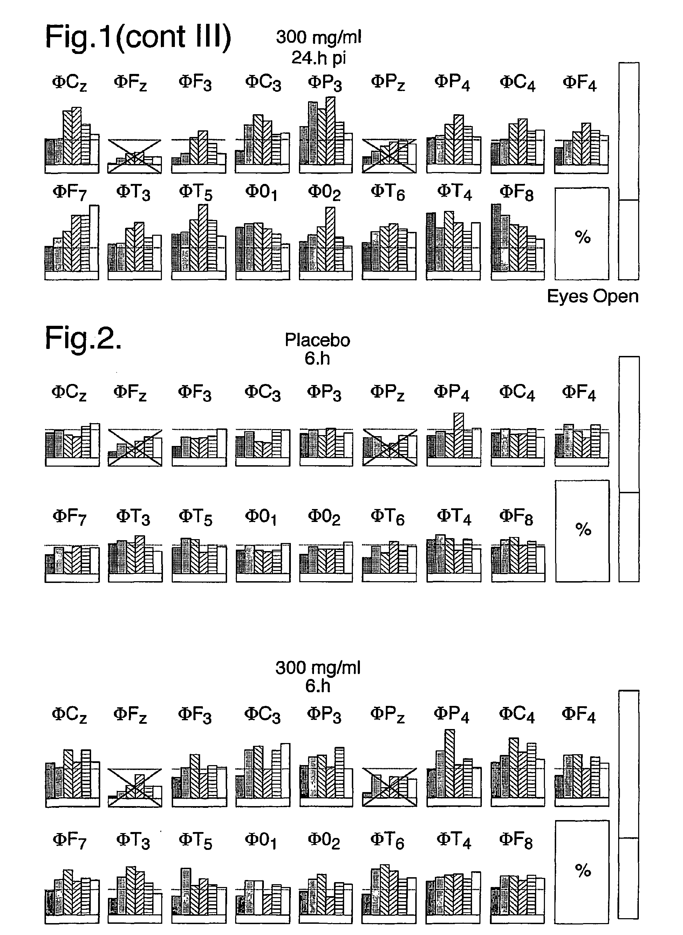

Figure 1: Documentation of changes at single electrode positions in percent of pre-dose values for each of the recording times: 6, 1.2 and 24 h during infusion of KTX 0101 (300 mg/kg iv infused over 24h) and 1 h and 24 post-infusion (pi). Bar graphs represent frequency ranges from delta (1st left column), theta (2^{nd} left), alpha1 (3^{rd} left), alpha2 (4^{th} left), beta1 (5^{th} left) and beta2 (right column). Cortical electrode positions are labeled as C for central, F for frontal, T for temporal, P for parietal, O for occipital. Even numbers refer to the right hemisphere, odd numbers to the left hemisphere. Results from the ΦFz and ΦPz electrode positions (crossed-out on the Figure) were not included because of unreliable outputs from them. Data are shown for the condition: "eyes open".

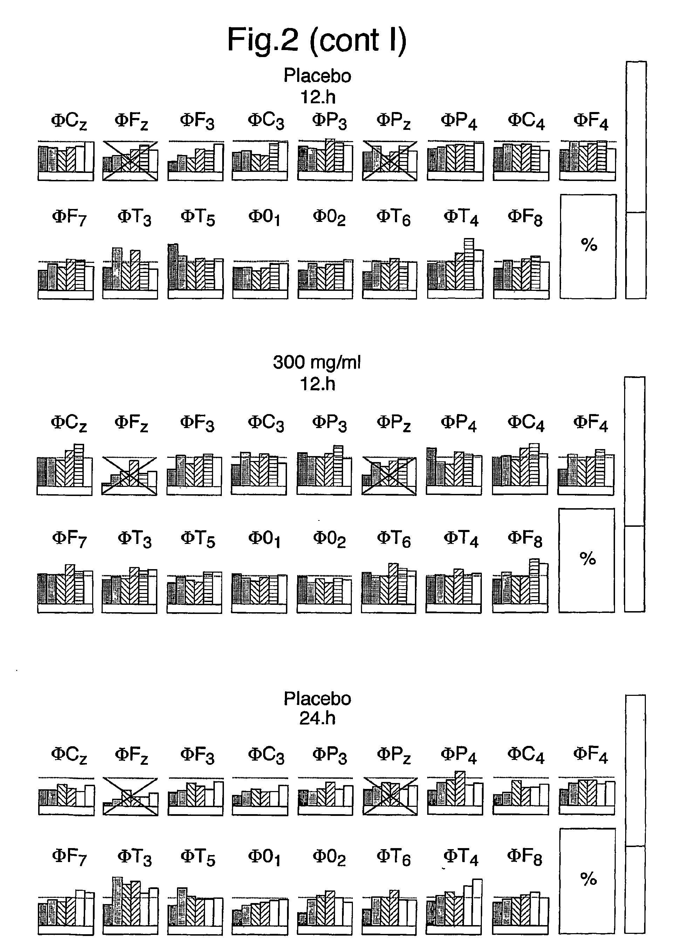

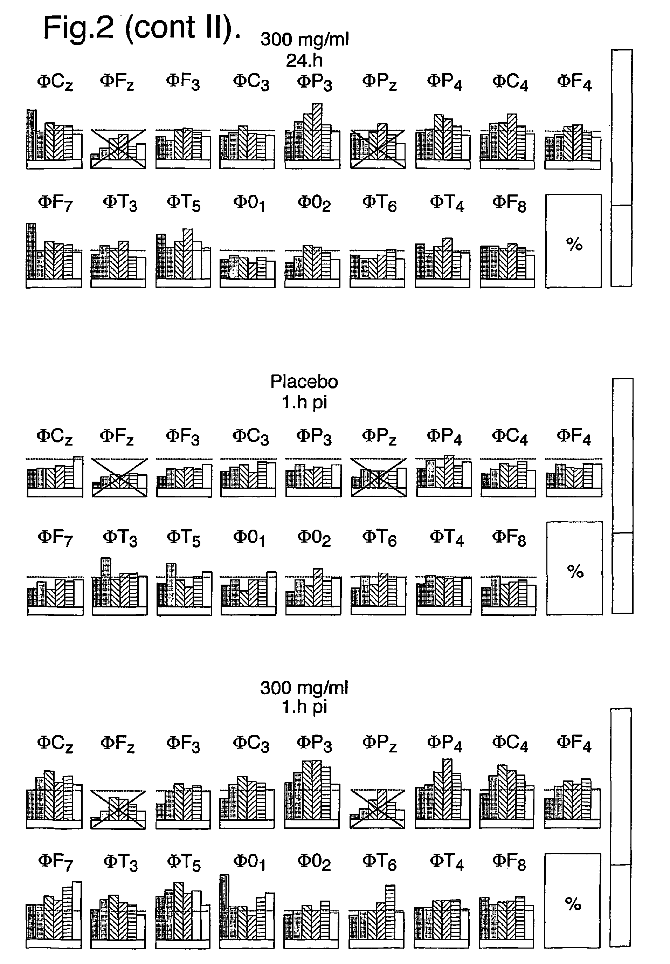

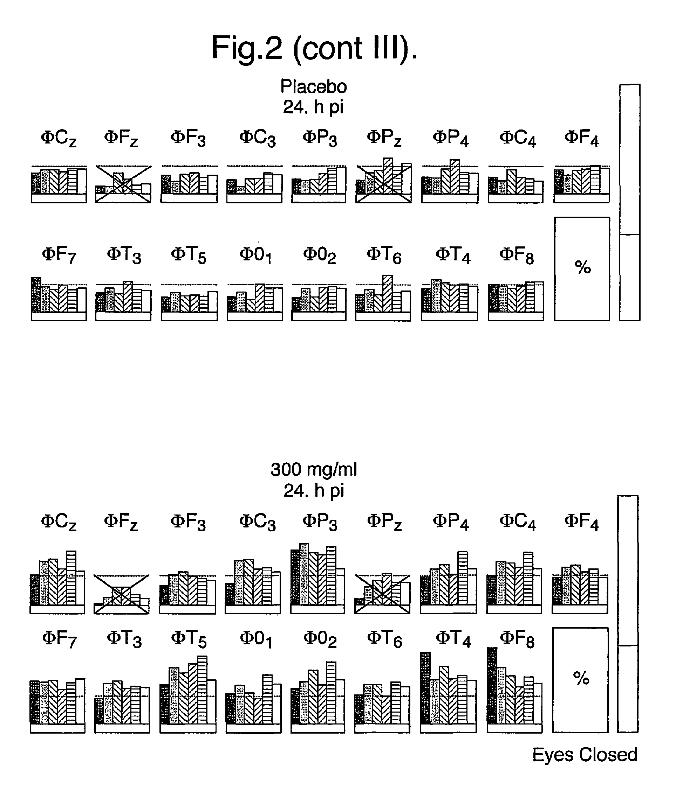

Figure 2: Documentation of changes at single electrode positions in percent of pre-dose values for each of the recording times: 6, 12 and 24 h during infusion of KTX 0101 (300 mg/kg iv infused over 24h) and 1 h and 24 post-infusion (pi). Bar graphs represent frequency ranges from delta (1^{st} left), theta (2^{nd} left), alpha1 (3^{rd} left), alpha2 (4^{th} left), beta1 (5^{th} left) and beta2 (right). Electrode positions are labeled as C for central, F for frontal, T for temporal, P for parietal, O for occipital. Even numbers refer to the right hemisphere, odd numbers to the left hemisphere. Results from the ΦFz and ΦPz electrode positions (crossed-out on the Figure) were not included because of unreliable outputs from them. Data are shown for the condition: "eyes closed".

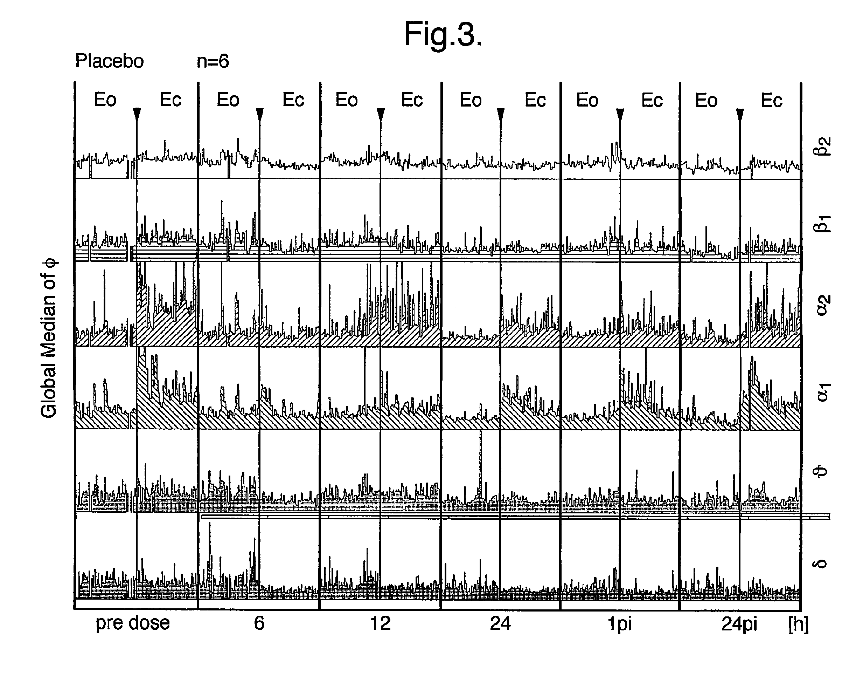

Figure 3: Time-course of recording periods during placebo administration. The infusion period for placebo was 24 h. Recordings were taken pre-dose, 6,12 and 24 h during infusion and 1 and 24 h post-infusion (pi). Recording periods consist of 5 minutes "eyes open" (Eo) followed by 5 minutes "eyes closed" (Ec) for each time-point. Global median of power is shown.

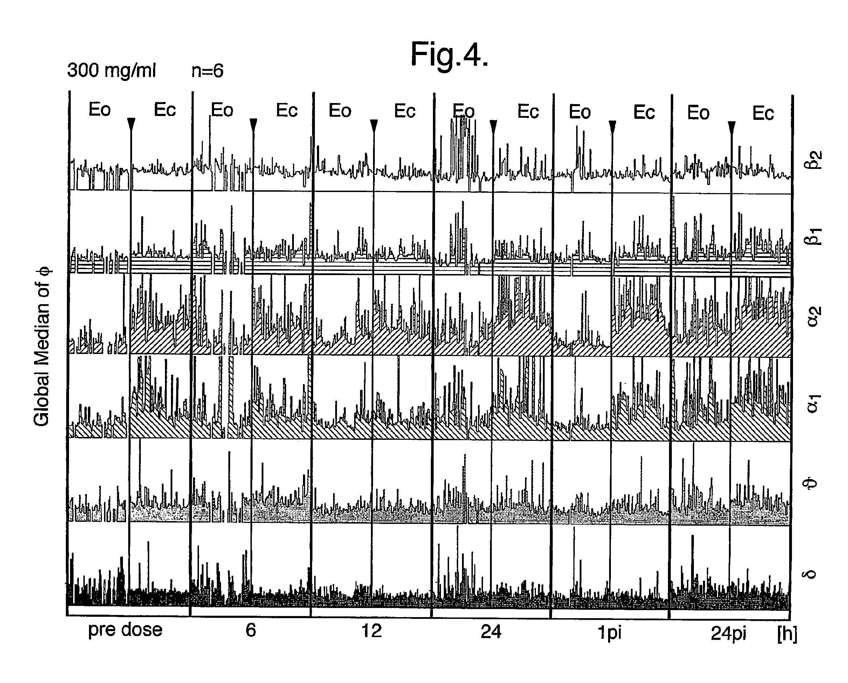

Figure 4: Time-course of recording periods during drug administration. Infusion period was 24 h. Recordings were taken pre-dose, 6h, 12 and 24 h during infusion and 1 and 24 h post-infusion (pi). Recording periods consist of 5 minutes "eyes open" (Eo) followed by 5 minutes "eyes closed" (Ec) for each time-point. Global median of power is shown.

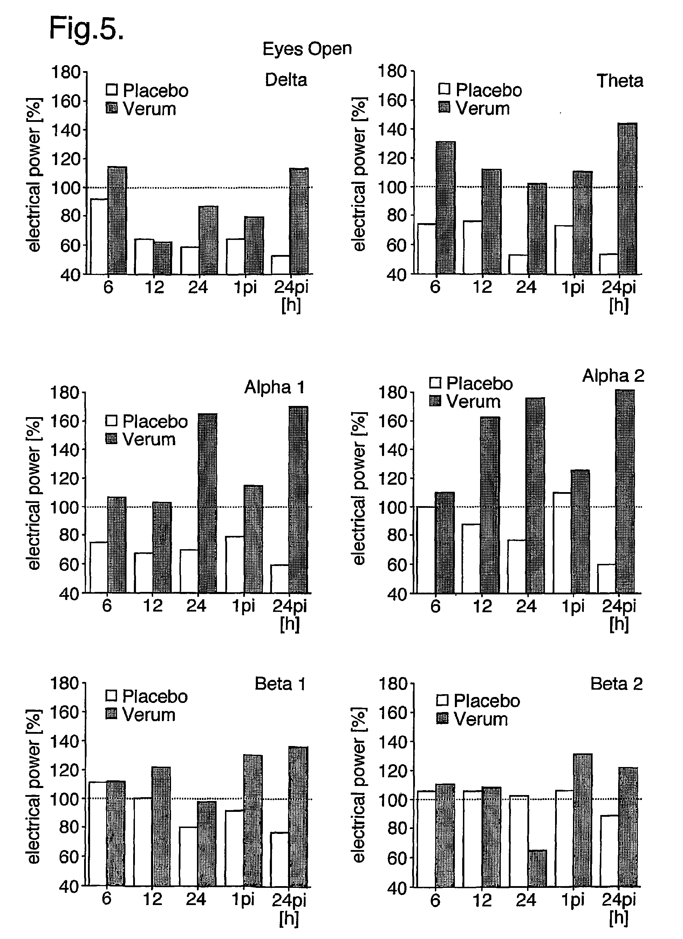

Figure 5: Documentation of electrical power changes at recording periods during and following placebo and KTX 0101 (300 mg/kg iv over 24h) infusion for the condition "eyes open". Pre-dose values were set to 100% (represented by dotted line). The effects of placebo are shown by the light shading in the histobars and those of KTX 0101 (300 mg/kg iv infused over 24h [denoted as Verum]) are shown by the dark shading. Each frequency range is shown separately from delta, through theta, alpha1, alpha2, beta1 and beta2. For definition of frequency ranges see Methods (below).

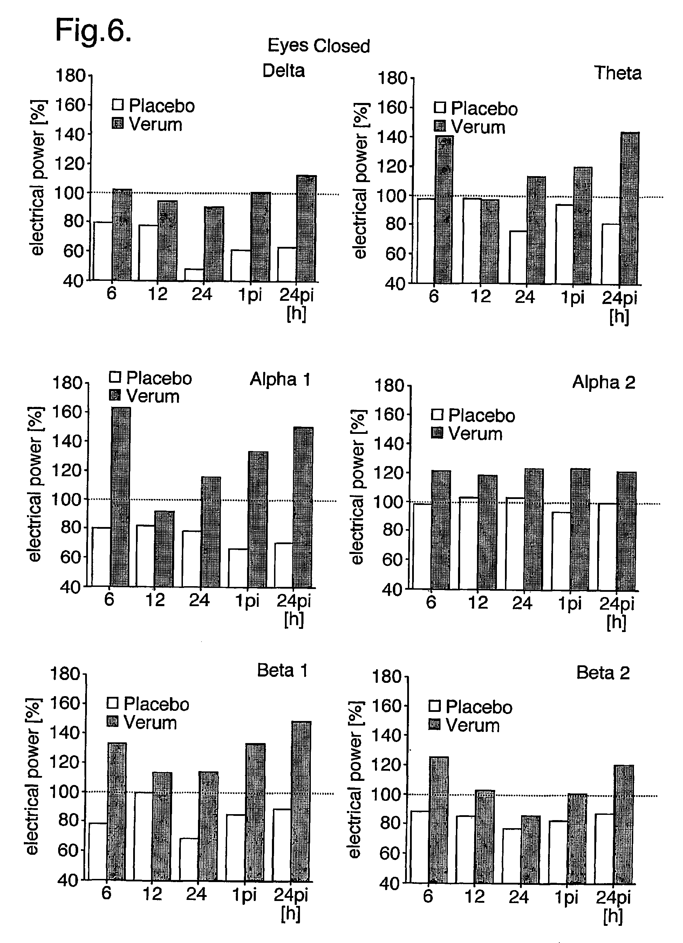

Figure 6: Documentation of electrical power changes at recording periods during and following placebo and KTX 0101 (300 mg/kg iv over 24h) infusion for the condition "eyes closed". Pre-dose values were set to 100% (represented by dotted line). The effects of placebo are shown by the light shading in the histobars and those of KTX 0101 (300 mg/kg iv infused over 24h [denoted as Verum]) are shown by the dark shading. Each frequency range is shown separately from delta, through theta, alpha1, alpha2, beta1 and beta2. For definition of frequency ranges see see Methods (below).

### METHODS.

Since functional changes of brain activity can most easily be accessed by recording electrical activity from the scalp, advanced EEG technology (CATEEM^{®}) was used to characterize the effects of KTX 0101 on the brain.

### Monitoring brain activity by quantitative EEG

Monitoring the electrical activity of the human brain has been a major challenge since the first report on the feasibility of its measurement by the German researcher Hans Berger in 1929 (Berger 1929). As early as 1932, he together with Dietsch suggested to use the mathematical approach of frequency analysis in order to quantitatively describe the information content of the recorded signals (Dietsch and Berger 1932). This idea had to await modern computer technologies available since the 1960's (Fink *et al* 1967) to perform the necessary calculations within a reasonable time. Since then an ever-increasing amount of literature describes changes of electrical activity of the brain in response to disease states, drug administration and behavioral states (Saletu and Grünberger 1988, Itil *et al* 1991, Itil and Itil 1995). Reflection of mental work on the topographical EEG was proven following this (Schober *et al* 1995).

### Study design

The study was designed to meet a number of objectives. The main objectives was to obtain safety and pharmacokinetic data which are reported separately. The other objective was to gain preliminary information on possible changes of electrical activity of the human brain since this activity is a very sensitive marker of possible actions of the drug on the brain.

Single rising doses of KTX 0101 or placebo were administered to groups of healthy male volunteers according to a pre-specified dose escalation schedule (see main report). Incremental doses were administered in a stepwise manner proceeding to each higher dose only if the drug was well tolerated and the criteria for stopping dosing had not been met. The last two cohorts obtained the highest doses of 300 mg/kg and consisted of enough volunteers to justify a preliminary evaluation of recorded EEG data in order to obtain information on the pharmacodynamic effects of the potential drug. After recording of pre dose values the infusion was started and continued for 24 hours. EEG recordings took place during the infusion (6, 12 and 24 h) and 1 and 24 h after the end of the infusion. Each recording period was performed under 5 minutes eyes open and 5 minutes of eyes closed condition.

### METHODOLOGY

### EEG-analysis

The EEG was recorded bipolarly from 17 surface electrodes according to the international 10/20 system with Cz as a physical reference electrode (Computer aided topographical electro-encephalo-metry: CATEEM^{®} from MediSyst GmbH, 35440 Linden, Germany), using an electrocap. The raw signals were amplified, digitized (2048 Hz/12 bit) and transmitted via fiber optical devices to the computer. The automatic artefact rejection of the CATEEM^{®}-System, which eradicates EEG-alterations caused by eyeblinks, swallows, respiration, ect. during the recording was automatically controlled and individually adjusted by the investigator. ECG and EOG were recorded in one channel each in order to facilitate detection of those signals superposing on to the EEG. The artefact rejection set-up was observed for about 5 minutes prior to the start of the recording to ensure, that all artefacts were correctly eliminated from further evaluation. For safety purposes the original raw data was saved on optical disk in order to allow re-evaluation of the artefact rejection mode if necessary. In these cases the experimental session was re-examined offline with a newly adapted rejection mode. The amount of rejected data was determined automatically and given in percent of total recording time. Nevertheless the entire recording and the computer-based automatic artefact rejection were continuously supervised and adjusted by a trained technician (Schober and Dimpfel, 1992). The data was recorded under two physiological conditions over a period of 5 minutes each (eyes open and eyes closed).

Using a Lagrange interpolation, signals from 82 additional virtual electrodes were calculated to provide high resolution topographical maps. The signals of all 99 electrode positions (17 real and 82 virtual) underwent the Fast Fourier Transformation (FFT) based on 4-second sweeps of data epochs (Hanning window). Data were analysed from 0.86 to 35 Hz using the CATEEM® software. In this software the resulting frequency spectra are divided into six frequency bands: delta (1.25 - 4.50 Hz), theta (4.75 - 6.75 Hz), alpha1 (7.00 - 9.50 Hz), alpha2 (9.75 - 12.50 Hz), beta1 (12.75 - 18.50 Hz) and beta2 (18.75 - 35.00 Hz). This frequency analysis is based on absolut spectral power values. Data acquisition and analysis were carried out simultanously and provided topographical maps displayed on-line on the computer screen. The resultant recordings from each time point were concatenated to form a single file for each administration (i.e. each study day) in order to present a continuous time course of drug effect. Data of the time course are presented as median over all 17 electrode positions (global median).

### 3.2 Raw data documentation and statistical analysis

Following a check of the raw data for optimal artifact rejection (new offline analysis was performed), the data was concatenated to give a single file for each subject containing all recording periods for eyes open and closed conditions. Subsequently, group files were built for each recording period and recording condition for documentation and statistical analysis.

Results are presented for each electrode position, as a time course of global median power and bar graphs showing the difference between placebo and active drug for each recording period. In order to analyse any changes induced by KTX 0101, the data from the pre-dose period was set to 100% and changes were calculated and depicted in relation to these values for the condition eyes open and closed separately. Values obtained for each recording period were averaged to give median values. The quartiles have not been depicted since statistical testing was performed for each recording period and frequency.

For statistical evaluation the non-parametric Wilcoxon-Whitney test was used though a partial cross-over design was used. This can be justified since only an explorative statistic evaluation was intended. At least 5 elements per group were evaluated using this statistical methodology (some subjects did not have a suitable recording). Data were successfully analysed for n=5-6 subjects. As it was a preliminary study in a small number of volunteers, the following statistical differences were considered to be of biological significance, viz P ≤ 0.20, P ≤ 0.10, P≤ 0.05 (80%, 90% and 95% probabilities, respectively, of a difference between placebo and drug effect)

### RESULTS

### Effect of intravenous 300 mg /kg on the electrical brain activity during eyes open.

Quantitative evaluation of EEG data was done by recording the electrical activity of the pre-dose phase for 5 minutes during the physiological condition eyes open and closed, respectively. Subsequent recording periods (5 min eyes open and 5 min eyes closed) were performed at 6, 12 and 24 hours during intravenous administration of KTX 0101 (300 mg/kg iv infused over a 24h period) and 1h and 24 h thereafter.

As documented in Fig. 1 the placebo infusion resulted in decreases of slow wave delta and theta power accompanied by some increases in fast frontal and temporal beta power. Under the condition of active drug an increase of delta and theta power as well as of alpha power is observed. These changes are most pronounced at 6 hours during the infusion, decreasing somewhat at the 12 h value but continued to be rather obvious during the rest of the recording time. Unfortunately there were some artefacts on the electrode positions Fz and Pz during the pre-dose time. Therefore these positions have been excluded from further quantitative analysis. Especially with respect to the parietal P3 and P4 electrodes we see firstly clear increases of delta and theta power at 6 h during infusion, then increases in alpha2 power at 12 h and finally alpha1 increases in addition at the end of infusion at 24 h. These effects decrease somewhat at 1 h after the end of infusion but are still observed extensively at 24 h after the end of infusion. Fig. 3 shows the time course of the changes for the median of 15 electrode positions (Fz and Pz were omitted because of artefacts during the pre-dose recording) for the placebo and active drug conditions, respectively. Averages of electrical power for each recording period in relation to pre-dose values are given in Fig. 5. As can be seen from the graphs the difference between placebo and active drug is largest for the theta, alpha1 and alpha2 power during the infusion period. There are still remarkable differences up to 24 h after end of the infusion.

### Effect of intravenous 300 mg/kg on the brain activity during_eyes closed

Quite similar changes were seen under the condition of eyes closed. There was some decrease of slow waves, especially during the later hours, but in general the recordings showed stabile conditions. In the presence of active drug, increases of electrical power could be observed for the 6 h time period, less for the 12 h period but consistently thereafter. These increases were seen mostly in the centro-parietal regions of the brain and were confined to theta, alpha1, alpha2 and beta 1 frequency ranges. A detailed statistical analysis is given in Table 1. Fig. 4 shows the time course of the changes for the median of 15 electrode positions (Fz and Pz were omitted because of artefacts during the pre-dose recording). Averages of electrical power for each recording period are given in Fig. 6. Essentially identical differences between placebo and active drug were seen under this condition of eyes closed. Statistical evaluation showed that the changes observed were highly significant at 6 h during the infusion but also with regard to the post-infusion period of 24 h. Details are given in Table 1.

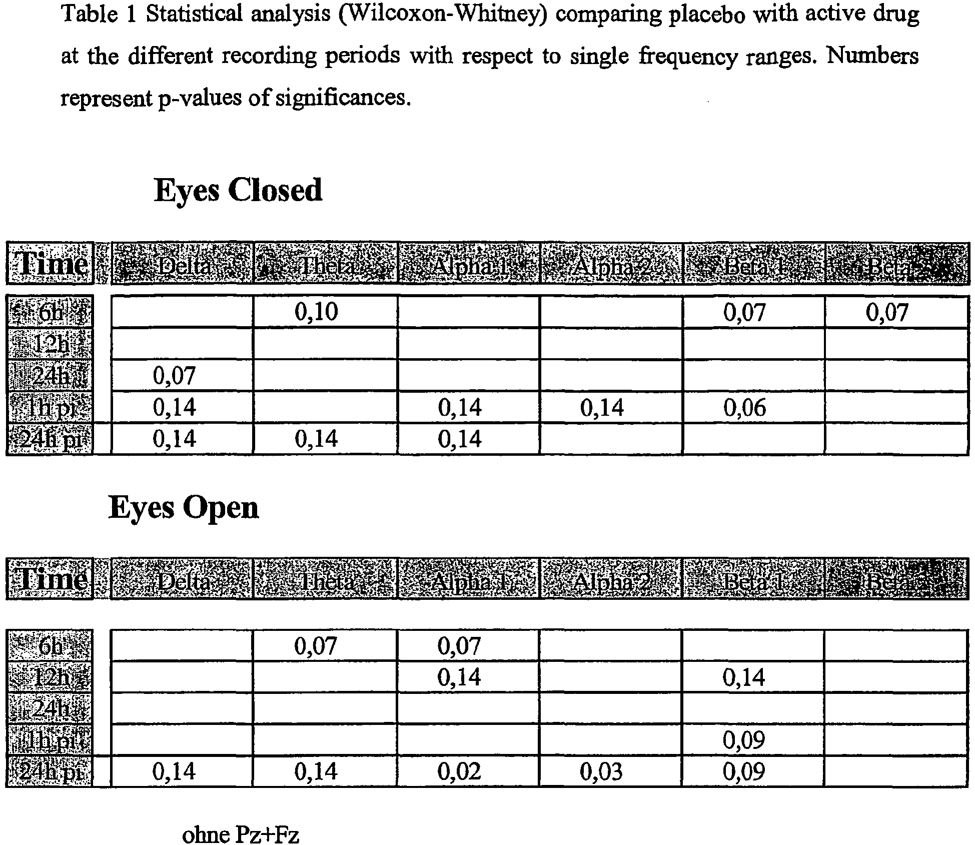

Thus differences between placebo and KTX 0101 could be observed mainly with respect to middle frequencies (theta, alpha and betal). Increases of the electrical power were seen in relation to pre-dose values only in the active drug cohort. The changes lasted longer than the duration of the infusion and could be traced up to 24 hours thereafter.

### 5. DISCUSSION

Since the electrical power within the theta and beta frequencies increases during the cooling of patients (see Kochs, 1995), these changes may be interpreted as indicative of a cytoprotective action of KTX 0101 in these subjects and this finding is entirely consistent with the known actions of the compound (see Smith et al, 2005). What was unexpected was to discover that KTX 0101 infusion evoked changes in the EEG power spectrum similar to those of drugs including barbiturates, opiates, benzodiazepines, and α₂-adrenoceptor agonists, which are in used both as premedications in surgical procedures and as agents to manage post-operative pain and stress as well as other complications. Thus, power increases in the beta range of varying degrees have been observed in rats after administration of sedative analgesics, including phenobarbital (barbiturate sedative, analgesic, anxiolytic, muscle-relaxant), diazepam (benzodiazepine sedative, anxiolytic, amnesic, muscle-relaxant), buprenorphine and morphine (opiate, narcotic analgesics) and flupertine (non-opiate analgesic) (see Dimpfel et al, 1986). These drug classes are all used as pre-medications in surgery and as agents to manage post-operative pain as well as other complications (see Tolksdorf et al, 1987; Drautz et al, 1991; Burkardt et al, 1997; Frank et al, 1999, 2002; Ornaque et al 2000). Combined increases in theta, alpha1,2 and beta1,2 power have been reported to occur in rats after administration of noradrenergic α₂-adrenoceptor agonists, eg metedomidine, guanfacine, clonidine, maxonidine and (-)lofexidine, (see Dimpfel and Schober, 2001). This class of drug has long been employed in the pre-surgical setting for its sedative, analgesic, anti-emetic and anaesthetic-sparing effects and post-surgically to prolong anaesthesia-induced analgesia and to reduce post-operative shivering (see Kulka et al, 1996; Oliver et al, 1999; El-Kerdawy et al 2000; Frank et al, 2002; Akbas et al, 2005). Lastly, combined increases in alpha1,2 and beta1,2 power have been reported to occur in rats after administration of general anaesthetics, eg halothane, desflurane, enflurane and isoflurothane (halogenated gaseous anaesthetics) and propofol (steroidal injectable anaesthetic), (see Dimpfel, 2003). When comparing the EEG effects induced by KTX 0101 to those of the drugs described above, the most marked similarity exists between its actions and those previously reported for the non-opiate analgesic, flupertine (Dimpfel et al, 1986), with strong similarities also to those of the α₂-adrenoceptor agonists, moxonidine and (-)lofexidine (Dimpfel and Schober, 2001) and the general anaesthetics, propofol and enflurane (Dimpfel, 2003).

Together, these changes in the EEG power spectra evoked by infusion of KTX 0101, which are present not only during the infusion period, but also for many hours thereafter, indicate that KTX 0101 has the unexpected ability to provide "stabilisation" to patients in the peri-surgical setting by virtue of its sedative, anxiolytic, anaesthetic-sparing and/or analgesic actions. KTX 0101 is not a pharmacological intervention because it produces its beneficial effects by providing a key substrate of physiological, mitochondrial oxidative phosphorylation, and therefore, it will not give rise to serious side-effects or adverse events that arise from drug-drug interactions that can arise with conventional agents, eg barbiturates, benzodiazepines, opiates or α₂-adrenoceptor agonists (see Kuchta and Goliembiewski, 2004).

### REFERENCES

Akbas M, Akbas H, Yegin A, Sahin N, Titiz TA (2005). Comparison of the effects of clonidine and ketamine added to ropivacaine on stress hormone levels and the duration of caudal analgesia. Paediatr Anaesth. 15:580-5.
Berger H (1929). Über das Elektroenzephalogramm des Menchen. Arch Psychiatr 87:527-570.
Burkhardt U, Wild L, Vetter B, Olthoff D (1997). Modulation of the stress response in children in the pre-operative preparation. Anaesthesist. 46:850-5. [in German].
Citerio G, Cormio M, Polderman KH (2004). Moderate hypothermia in traumatic brain injury: results of clinical trials. Minerva Anestesiol. 70:213-8. [in Italian].
Dietsch G, Berger H (1932). Fourier Analyse von Elektroenzephalogrammen des Menschen. Pflügers Arch 230:106-112.
Schober F, Dimpfel W (1992). Relation between psychometric tests and quantitative topographic EEG in pharmacology. Int J Clin Pharmacol Ther Toxicol. 30:428-30.
Dimpfel W, Schober F (2001). Norepinephrine, EEG theta waves and sedation. Brain Pharmacol. 1:89-97.
Dimpfel W, Spuler M, Nickel B (1986). Radioelectroencephalography (Tele-Stereo-EEG) in the rat as a pharmacological model to differentiate the central action of flupirtine from that of opiates, diazepam and phenobarbital. Neuropsychobiology. 16:163-8.
Dimpfel W (2003). Preclinical data base of pharmaco-specific rat EEG fingerprints (tele-stereo-EEG). Eur J Med Res. 8:199-207.
Drautz M, Feucht A, Heuser D (1991). A comparative study of the efficacy and tolerance of dipotassium clorazepate and flunitrazepam for oral premedication. Anaesthesist. 40:651-60. [in German].
El-Kerdawy HM, Zalingen EE, Bovill JG (2000). The influence of the α2-adrenoceptor agonist, clonidine, on the EEG and on the MAC of isoflurane. Eur J Anaesthesiol. 17:105-10.
Fink M, Itil TM, Shapiro DM (1967). Digital computer analysis of the human EEG in psychiatric research. Compr Psychiatry. 8:521-38.
Frank T, Thieme V, OlthoffD (1999). Pre-operative clonidine comedication within the scope of balanced inhalation anesthesia with sevoflurane in oral surgery procedures. Anaesthesiol Reanim. 24:65-70. [in German].
Frank T, Wehner M, Heinke W, Schmadicke I (2002). Clonidine vs. midazolam for premedication - comparison of the anxiolytic effect by using the STAI-test. Anasthesiol Intensivmed Notfallmed Schmerzther. 37:89-93. [in German].
Galasko CS, Courtenay PM, Jane M, Stamp TC (1985). Trial of oral flupirtine maleate in the treatment of pain after orthopaedic surgery. Curr Med Res Opin. 9:594-601.
Itil TM, Mucci A, Eralp E (1991). Dynamic brain mapping methodology and application. Int J Psychophysiol. 10:281-91.
Itil TM, Itil KZ (1995). Quantitative EEG brain mapping in psychotropic drug development, drug treatment selection and monitoring. Am J Ther. 2:359-367.
Kochs E (1995). Electrophysiological monitoring and mild hypothermia. J Neurosurg Anaesthesiol. 7:222-8.
Kuchta A, Golembiewski J (2004). Medication use in the elderly patient: focus on the perioperative/perianesthesia setting. J Perianesth Nurs. 19:415-24.
Kulka PJ, Tryba M, Zenz M (1996). Preoperative α2-adrenergic receptor agonists prevent the deterioration of renal function after cardiac surgery: results of a randomized, controlled trial. Crit Care Med. 24:947-52.
Lasater M (2005). The role of thermoregulation in cardiac resuscitation. Crit Care Nurs Clin North Am. 17:97-102, xii.
Martin E, Ramsay G, Mantz J, Sum-Ping ST (2003). The role of the α2-adrenoceptor agonist dexmedetomidine in postsurgical sedation in the intensive care unit. J Intensive Care Med. 18:29-41.
Moore RA, Bullingham RE, Simpson S, O'Sullivan G, Evans PJ, McQuay HJ, Lloyd JW (1983). Comparison of flupirtine maleate and dihydrocodeine in patients following surgery. Br J Anaesth. 55:429-32.
Oliver MF, Goldman L, Julian DG, Holme I (1999). Effect of mivazerol on perioperative cardiac complications during non-cardiac surgery in patients with coronary heart disease: the European Mivazerol Trial (EMIT). Anesthesiology. 91:951-61.
Ornaque I, Carrero E, Villalonga A, Roux C, Salvador L (2000). Study of presurgical anxiety in urologic, gynecologic and ophthahnologic surgery as a function of the administration or non-administration of anxiolytic premedication. Rev Esp Anestesiol Reanim. 47:151-6. [in Spanish].
Pace MC, Palagiano A, Pace L, Passavanti MB, Iannotti M, Sorrentino R, Aurilio C (2004). Sedation in gynaecologic oncology day surgery. Anticancer Res. 24:4109-12.
Riethmuller-Winzen H (1987). Flupirtine in the treatment of post-operative pain. Postgrad Med J. 63 (Suppl 3):61-5.
Saletu B, Grünberger J (1988). Drug profiling by computed electroencephalography and brain maps with special consideration of sertraline and its psychmetric effects. J Clin Psychiatr. 49 (Suppl):59-71.
Schober F, Schellenberg R, Dimpfel W (1995). Reflection of mental exercise in the dynamic quantitative topographical EEG. Neuropsychobiology 31:98-112.
Smith SL, Heal DJ, Martin KF (2005). KTX 0101: a potential metabolic approach to cytoprotection in major surgery and neurological disorders. CNS Drug Rev. 11:113-40.
Tolksdorf W, Gerlach C, Hartung M, Hettenbach A. (1987). Midazolam and pethidine/promethazine for intramuscular premedication. Anaesthesist. 36:275-9. [in German].
Wagner KR, Zuccarello M (2005). Local brain hypothermia for neuroprotection in stroke treatment and aneurysm repair. Neurol Res. 27:238-45.

## Claims

1. A ketogenic material selected from (R)-3-hydroxybutyrate, its salts_and an (R)-3-hydroxybutyrate containing material selected from oligomeric (R)-3-bydroxybuytrate and esters of (R)-3-hydroxybuytrate with glycerol or (R)-butan-1,3-diol, for use in treating a patient to provide stabilisation during surgery **characterised in that** treatment is for provision of sedation and the treatment is with a dose of material of 5-5000 mg/kg body weight and raises the total concentration of acetoacetate and (R)-3-hydroxybutyrate in the blood of the subject patient to between 0.1 and 30mM.

2. A ketogenic material for the use as claimed in Claim 1 **characterised in that** the treatment is a perisurgical adjunct to surgery.

3. A ketogenic material for the use as claimed in Claim 1 or Claim 2 **characterised in that** the surgery is performed under general or local anaesthesia.

4. A ketogenic material for the use as claimed in any one of the preceding claims **characterised in that** the treatment is anaesthetic-sparing and/or analgesic and/or anxiolytic.

5. A ketogenic material for the use as claimed in any one of the preceding claims **characterised in that** the surgery is selected from the group consisting of removal or section of tumours, removal of redundant organs such as lymph nodes and appendix, cardio-thoracic, gynaecological, urological, opthalmological, cosmetic and orthopaedic surgery, neurosurgery and organ transplantation.

6. A ketogenic material for the use as claimed in any one of the preceding claims wherein the surgery is selected from the group consisting of open heart surgery and joint and bone surgery.

7. A ketogenic material for the use as claimed in Claim 1 wherein the total concentration of acetoacetate and (R)-3-hydroxybutyrate in the blood is between 0.5 and 15mM.

8. A ketogenic material for the use as claimed in Claim 7 **characterised in that** the total concentration of acetoacetate and (R)-3-hydroxybutyrate in the blood is raised to between 1 and 10mM.

9. A ketogenic material for the use as claimed in Claim 8 **characterised in that** the total concentration of acetoacetate and (R)-3-hydroxybutyrate in the blood is raised to between 3 and 8mM.

10. A ketogenic material for the use as claimed in any one of the preceding claims **characterised in that** the ketogenic material is selected from sodium (R)-3-hydroxybutyrate, multimers of (R)-3-hydroxybutyrate, triglyceride of (R)-3-hydroxybutyrate and triolide of (R)-3-hydroxybutyrate.

## Patentansprüche

1. Ketogenes Material ausgewählt aus (R)-3-Hydroxybutyrat, dessen Salze und einem (R)-3-Hydroxybutyrat enthaltenden Material ausgewählt aus oligomerem (R)-3-Hydroxybutyrat und Estern von (R)-3-Hydroxybutyrat mit Glycerin oder (R)-Butan-1,3-diol, zur Verwendung bei der Behandlung eines Patienten, um Stabilisierung während einer Operation bereitzustellen, **dadurch gekennzeichnet, dass** die Behandlung für die Bereitstellung der Sedierung vorgesehen ist und die Behandlung mit einer Materialdosis von 5-5000 mg/kg Körpergewicht durchgeführt wird und die gesamte Konzentration von Acetoacetat und (R)-3-Hydroxybutyrat im Blut des zu behandelnden Patienten auf zwischen 0,1 und 30 mM erhöht.

2. Ketogenes Material für die Verwendung wie beansprucht in Anspruch 1, **dadurch gekennzeichnet, dass** die Behandlung eine peri-operative Ergänzung zur Operation darstellt.

3. Ketogenes Material für die Verwendung wie beansprucht in Anspruch 1 oder Anspruch 2, **dadurch gekennzeichnet, dass** die Operation unter Voll- oder Lokalanästhesie durchgeführt wird.

4. Ketogenes Material für die Verwendung wie beansprucht in einem der vorangegangenen Ansprüche, **dadurch gekennzeichnet, dass** die Behandlung Anästhetikum-sparend und/oder analgetisch und/oder anxiolytisch ist.

5. Ketogenes Material für die Verwendung wie beansprucht in einem der vorangegangenen Ansprüche, **dadurch gekennzeichnet, dass** die Operation ausgewählt ist aus der Gruppe bestehend aus Entfernung oder Sektion von Tumoren, Entfernung von redundanten Organen wie Lymphknoten und Blinddarm, kardiothorakalen, gynäkologischen, urologischen, ophthalmologischen, kosmetischen und orthopädischen Operationen, Neurochirurgie und Organtransplantation.

6. Ketogenes Material für die Verwendung wie beansprucht in einem der vorangegangenen Ansprüche, wobei die Operation ausgewählt ist aus der Gruppe bestehend aus einer Operation am offenen Herzen und Gelenk- und Knochenoperationen.

7. Ketogenes Material für die Verwendung wie beansprucht in Anspruch 1, wobei die gesamte Konzentration an Acetoacetat und (R)-3-Hydroxybutyrat im Blut bei zwischen 0,5 und 15 mM beträgt.

8. Ketogenes Material für die Verwendung wie beansprucht in Anspruch 7, **dadurch gekennzeichnet, dass** die gesamte Konzentration von Acetoacetat und (R)-3-Hydroxybutyrat im Blut auf zwischen 1 und 10 mM erhöht wird.

9. Ketogenes Material für die Verwendung wie beansprucht in Anspruch 8, **dadurch gekennzeichnet, dass** die gesamte Konzentration von Acetoacetat und (R)-3-Hydroxybutyrat im Blut auf zwischen 3 und 8 mM erhöht wird.

10. Ketogenes Material für die Verwendung wie beansprucht in einem der vorangegangenen Ansprüche, **dadurch gekennzeichnet, dass** das ketogene Material ausgewählt ist aus Natrium (R)-3-Hydroxybutyrat, Multimeren von (R)-3-Hydroxybutyrat, dem Triglycerid von (R)-3-Hydroxybutyrat und dem Triolid von (R)-3-Hydroxybutyrat.

## Revendications

1. Matériau cétogène sélectionné parmi le (R)-3-hydroxybutyrate, ses sels et un matériau contenant du (R)-3-hydroxybutyrate sélectionné parmi le (R)-3-hydroxybutyrate oligomérique et des esters de (R)-3-hydroxybutyrate avec du glycérol ou du (R)-butan-1,3-diol, destiné à être utilisé pour traiter un patient afin d'obtenir une stabilisation pendant une intervention chirurgicale, **caractérisé en ce que** le traitement permet d'obtenir une sédation et le traitement est réalisé avec une dose de matériau de 5-5000 mg/kg de poids corporel et augmente la concentration totale de l'acétoacétate et du (R)-3-hydroxybutyrate dans le sang dudit patient dans une plage comprise entre 0,1 et 30 mM.

2. Matériau cétogène destiné à être utilisé selon la revendication 1, **caractérisé en ce que** le traitement est un traitement périchirurgical complémentaire d'une intervention chirurgicale.

3. Matériau cétogène destiné à être utilisé selon la revendication 1 ou la revendication 2, **caractérisé en ce que** l'intervention chirurgicale est réalisée sous anesthésie générale ou locale.

4. Matériau cétogène destiné à être utilisé selon l'une quelconque des revendications précédentes, **caractérisé en ce que** le traitement est un traitement d'épargne anesthésique et/ou analgésique et/ou anxiolytique.

5. Matériau cétogène destiné à être utilisé selon l'une quelconque des revendications précédentes, caractérisé en ce l'intervention chirurgicale est sélectionnée dans le groupe consistant en l'exérèse ou la résection de tumeurs, l'exérèse d'organes redondants comme les ganglions lymphatiques et l'appendice, une intervention chirurgicale cardiothoracique, gynécologique, urologique, ophtalmologique, cosmétique et orthopédique, la neurochirurgie et une transplantation d'organe.

6. Matériau cétogène destiné à être utilisé selon l'une quelconque des revendications précédentes, où l'intervention chirurgicale est sélectionnée dans le groupe consistant en une chirurgie à coeur ouvert et une chirurgie articulaire et osseuse.

7. Matériau cétogène destiné à être utilisé selon la revendication 1, où la concentration totale de l'acétoacétate et du (R)-3-hydroxybutyrate dans le sang est comprise entre 0,5 et 15 mM.

8. Matériau cétogène destiné à être utilisé selon la revendication 7, **caractérisé en ce que** la concentration totale de l'acétoacétate et du (R)-3-hydroxybutyrate dans le sang est augmentée à une valeur comprise entre 1 et 10 mM.

9. Matériau cétogène destiné à être utilisé selon la revendication 8, **caractérisé en ce que** la concentration totale de l'acétoacétate et du (R)-3-hydroxybutyrate dans le sang est augmentée à une valeur comprise entre 3 et 8 mM.

10. Matériau cétogène destiné à être utilisé selon l'une quelconque des revendications précédentes, **caractérisé en ce que** le matériau cétogène est sélectionné parmi le (R)-3-hydroxybutyrate de sodium, des multimères de (R)-3-hydroxybutyrate, un triglycéride de (R)-3-hydroxybutyrate et un triolide de (R)-3-hydroxybutyrate.
